# EUROPEAN PATENT APPLICATION

(11) **EP 1 118 338 A2**
(43) Date of publication of application: **25.07.2001**
(21) Application number: 01300421.3
(22) Date of filing: 18.01.2001
(51) Int. Cl.: A61L 9/12

(54) **Air freshener system utilizing breakable cartridges**

(30) Priority: 18.01.2000 US 483991
(71) Applicant: STEINER COMPANY, INC., Chicago Illinois 60611 (US)
(72) Inventor: Kauzlarich, John, Darien, Illinois 60561 (US); Phillips, Brian, Crest Hill, Illinois 60435 (US); Voth, A. J., St. Charles, Illinois 60174 (US); McKinnell, Martin, DeKalb, Illinois 60115 (US)
(74) Representative: Newby, Martin John

(57) **Abstract**

A self-contained battery powered air freshener (10) including a housing (12,14) having an inlet portion through which ambient air is drawn into the housing be means of a battery powered fan (30). The air is passed about a quantity of vaporizable material (35) contained within the housing, and then discharged therefrom into the ambient air carrying the vaporized portion of the product therewith. One or more replaceable breakable cartridges (36), containing air freshening material, are positionable within a magazine (40) within the housing, the magazine (40) providing a support well (45) for supporting a replaceable battery (42) such that the deodorizing material and battery may be separately replaced when either of these items has become expended. The air freshener is released by breaking the breakable cartridges (36) inserted within the housing. The consumer can customize the strength of the air freshener by determining the quantity of cartridges to place in the housing and can customize the scent by providing differently scented cartridges in the same housing.

## Description

### Background of the Invention

This invention relates in general to air freshening devices and, in particular, to a self-contained air freshening device which includes breakable air freshening cartridges that are substantially impermeable to the air freshener contained therein when unbroken and permeable to the air freshener when broken.

More specifically, but without restriction to the particular embodiment and/or use which is shown and described for purposes of illustration, this invention relates to a self-contained air freshener apparatus utilizing one or more replaceable breakable cartridges comprising a breakable ampoule containing a vaporizable material. The ampoule is substantially impermeable to the vaporizable material while the ampoule is intact and the vaporizable material is released when the ampoule is broken. A permeable membrane preferably surrounds the ampoule and limits the rate of vaporization of the air freshener when the ampoule is broken. A protective covering can be provided between the permeable membrane and the ampoule to protect the membrane from abrasive surfaces of a broken ampoule. The protective covering also keeps the pieces of the broken ampoule in a single package to make replacement easy.

Various types of replaceable air freshening cartridges have been utilized for placement within, or about, a stream of air to distribute the air freshener. Typically, such cartridges contain an air freshener that can be vaporized, either by evaporation or sublimation. To this end, cartridges such as disclosed in U.S. Patent Nos. 4,743,406; 4,491,224 and 5,126,078 have been developed. While these cartridges have been found to be very useful, they can still be improved upon.

In U.S. Patent No. 4,743,406, an apparatus is disclosed which utilizes a relatively bulky replaceable cartridge that comprises a quantity of vaporizable material in a generally concentric shape, airways for air to flow about both the interior and exterior surfaces of the cylindrical material, and a battery power source replaceably mounted within the cartridge. In U.S. Patent No. 4,931,224, another relatively bulky replaceable cartridge is disclosed that includes a packet of vaporizable material and an area that carries a battery. In U.S. Patent No. 4,931,224, another relatively bulky replaceable cartridge is disclosed that includes a felt cylindrical body impregnated with an air freshener and an area that carries a battery.

In each of the devices, the entire bulky unitary cartridge are normally replaced with a completely new cartridge to provide a fresh quantity of air freshener. However, each of these bulky cartridges utilize a relatively large quantity of replaceable materials that must be disposed of when the air freshener is depleted. These replaceable cartridges are largerthan necessary and wasteful of raw materials, especially compared to the amount of air fresher that they contain. The use of such large replaceable bodies for the cartridges can increase the costs of manufacturing, packaging, delivery, warehousing, and disposal.

These prior cartridges can also require packaging designed to contain the air freshener to prevent vaporization prior to actual use. This packaging must be removed prior to use and can be a source of confusion for personnel not familiar with the product. The special packaging can increase the costs of manufacturing and disposal.

These prior cartridges are available in predetermined sizes that do not allow a user to customize the strength of the air freshener used in a given application. If various scents were available, the manufacturer produced different cartridges for every different scent available to the consumer.

The present invention is constructed such that one, and preferably multiple, replaceable breakable cartridges are provided and loaded into a reusable magazine that is inserted into an air freshening apparatus. The replaceable cartridges are preferably impermeable to the air freshener until the cartridge is broken. A battery is inserted in and carried by the reusable magazine and the magazine is twisted causing one or more surfaces to contact and break the breakable cartridge. In another form, insertion of the battery can be utilized to cause the cartridges to break and release the air freshener.

Because multiple replaceable cartridges fit in each air freshening device, the user can determine the optimal number of cartridges to provide for each application. For example, more cartridges can be used when an extra quantity of air freshener is desired or to reduce the amount of servicing required by prolonging the amount of time before all of the air freshener is spent. Likewise, fewer cartridges can be used for less severe applications, for minimizing the strength of scent of the air freshener, or to minimize wasting the air freshener. The consumer can customize scents by using different scented cartridges in the same air freshening apparatus to obtain a custom scent. Likewise, a manufacturer can package differently scented cartridges together to provide customers with a wider variety of scents while minimizing the number of different cartridges that need to be manufactured and kept in inventory. Similarly, the different scented cartridges can be individually packaged and guides provided that suggest various combinations of scents.

### Summary of the Invention

It is, therefore, an object of this invention to improve self-contained air freshening devices.

Another object of this invention is to utilize a separate expendable and replaceable air freshening cartridge that decreases the quantity of disposable material used.

A further object of this invention is to provide a replaceable air freshening cartridge that can be used in single or multiple groups to vary the amount of air freshener used in an air freshening apparatus.

A further object of this invention is to provide a replaceable air freshening cartridge that can be used in multiple groups to allow for customization of scents by mixing and matching different cartridges in an air freshening device.

A further object of this invention is to provide a replaceable air freshening cartridge that lengthens cartridge shelf-life by preventing air freshener loss until an air freshener ampoule is broken.

Yet another object of this invention is to facilitate ease of servicing and replacement of expendable components.

These and other objects are attained in accordance with the present invention wherein there is provided a self-contained battery-powered room air freshener including a housing having an inlet portion through which ambient air is drawn into the housing by means of a battery powered fan. The air is passed about a quantity of vaporizable material contained within the housing, and then discharged therefrom into the ambient air carrying the vaporized portion of the product therewith. One or more replaceable breakable cartridges, containing air freshening material, are positionable within a magazine within the housing, the magazine provides a support well for supporting a replaceable battery such that the deodorizing material and battery may be separately replaced when either of these items has become expended. The air freshener is released by breaking the breakable cartridges inserted within the housing. The consumer can customize the strength of the air freshener by determining the quantity of cartridges to place in the housing and can customize the scent by providing different scented cartridges in the same housing.

### Brief Description of the Drawings

Further objects of the invention together with additional features contributing thereto and advantages accruing therefrom will be apparent from the following description of a preferred embodiment of the invention which is shown in the accompanying drawings with like reference numerals indicating corresponding parts throughout, wherein:
FIG. 1 is a cross-sectional view of an assembled self-contained deodorizing apparatus in accordance with the invention;
FIG. 2 is an exploded view of a first embodiment of the reusable magazine, the replaceable cartridges, and the battery used in the air deodorizing apparatus of FIG. 1;
FIG. 3 is a cross sectional view of the first form of reusable magazine with replaceable cartridges inserted therein;
FIG. 4 is a cross-sectional view of the reusable magazine of FIG. 3 with the insert rotated with respect to the sleeve portion;
FIG. 5 is a cross-sectional view of the reusable magazine shown in FIG. 3 along the lines 5-5;
FIG. 6 is a cross-sectional view of the apparatus shown in FIG. 4 along the lines 6-6;
FIG. 7 is a perspective view of a second embodiment of the reusable magazine used in the air deodorizing apparatus of FIG. 1;
FIG. 8 is an exploded view of the magazine of FIG. 7 including replaceable cartridges and a battery;
FIG. 9 is a cross sectional view of the reusable magazine of FIG. 7 along lines 9-9;
FIG. 10 is a cross-sectional view of the reusable magazine of FIG. 9 with replaceable cartridges inserted therein;
FIG. 11 is a cross-sectional view of the reusable magazine shown in FIG. 10 with the battery inserted therein;
FIG. 12 is the cross-sectional view of the apparatus shown in FIG. 11 along lines 12-12;
FIG. 13 is a perspective view of a replaceable cartridge; and
FIG. 14 is a cross-section view of the replaceable cartridge of FIG. 13 along lines 14-14.
FIG. 15 is a plan view of a third embodiment of the reusable magazine, the replaceable cartridges, and the battery used in the air deodorizing apparatus of FIG. 1;
FIG. 16 is an exploded perspective view of the reusable magazine, the replaceable cartridges, and the battery of FIG. 15;

### Description of the Preferred Embodiment

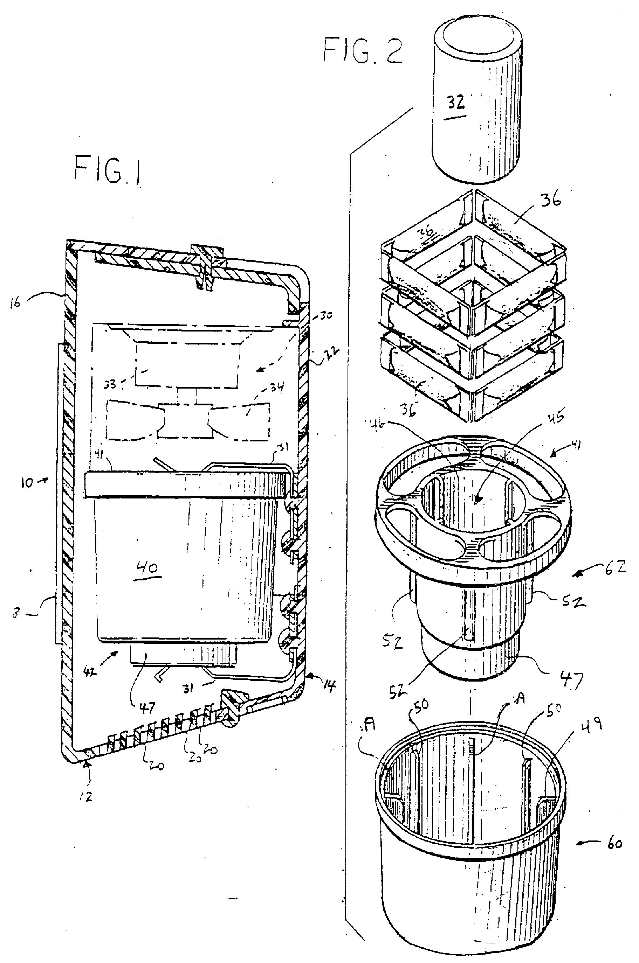

Referring now to FIG. 1, there is illustrated a self-contained air deodorizing device 10 which is formed in a substantially rectangular shape. In one form, deodorizing device 10 comprises a two-part housing including a mounting section 12 adapted to secure the device to a vertical wall and a closure section 14 which, when connected to the mounting section 12, together form a box-shaped enclosure. The mounting section 12 includes a rear wall portion 16 having a pair of openings (not shown) formed therein by which the mounting section 12 may be secured to a vertical surface. Alternatively, the mounting section can be attached to a wall by means of a pressure-sensitive adhesive-backed foam tape 18.

The lowermost portion of the mounting section 12 includes a plurality of slots 20 forming a discharge grille through which air is discharged from the deodorizing device. A plurality of slots (not shown) in the front wall 22 form an inlet grille through which ambient air may be drawn into the deodorizer.

In order to generate the flow of ambient air through the deodorizer housing, a battery powered electric motor driven fan assembly 30 is provided in the closure section 14. The motor and fan assembly 30 is preferably located adjacent to the air inlet slots (not shown) for drawing air into the unit. A two-piece bracket 31 is secured to the interior of a front wall 22 of the closure section 14 and functions to complete a circuit, including a battery 32 (see FIGS. 2, 4, 8, & 11), when electrically coupled between the bracket and an electric battery-powered motor 33 of the motor and fan assembly.

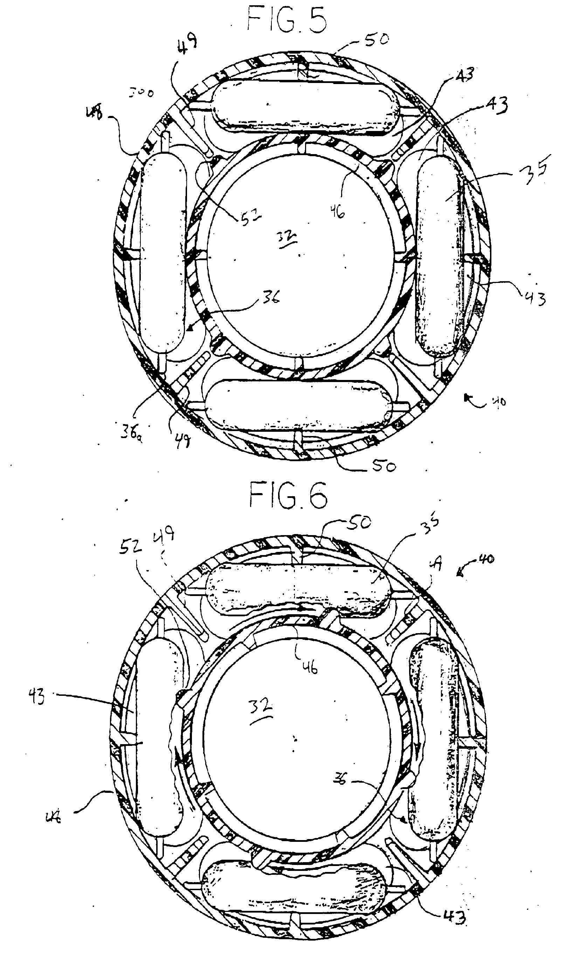

The motor 33 is preferably of a type which is operable in one direction but may be operable in either a forward or a reverse direction of rotation depending upon the polarity coupling of the battery 32 in the bracket 31. Normally, the motor and fan assembly 30 directs air flow from the inlet (not shown) on front wall 22 across a vaporizable material 35 (see FIGS. 5, 6 & 12) contained within a reusable magazine 40. While the motor and fan assembly 30 is shown above the reusable magazine 40, the motor and fan assembly may be reversed with the motor being positioned between the fan assembly and the reusable magazine.

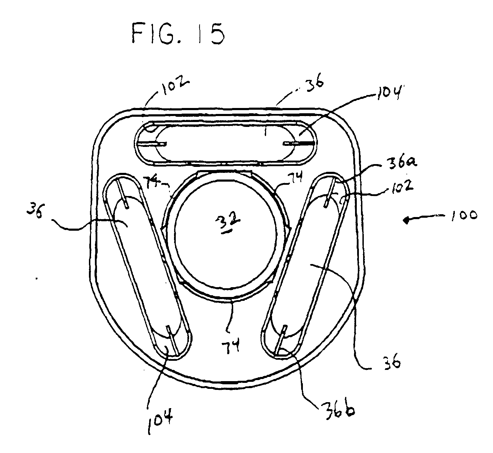

The vaporizable material 35 is preferably an air freshener or deodorizer in a liquid, sol, or gel form. However, another form, such as a solid, could also be used. As best illustrated in FIGS. 13 and 14, vaporizable material 35 is preferably contained within a replaceable breakable air freshening cartridge 36 that is removably inserted within reusable magazine 40. In a preferred form, breakable air freshening cartridge 36 is impermeable to the vaporizable material 35 when breakable cartridge 36 is unbroken or whole. One such suitable impermeable and breakable form is an elongated glass container, such as glass ampoule 37, however a suitable breakable plastic could easily be substituted. Another suitable form is an ampoule having a first condition in which the vaporizable material is enclosed and prevented from vaporizing to the atmosphere and a second condition in which the ampoule allows the vaporizable material to escape to the atmosphere.

A protective cover of any suitable material, such as for instance, a leisure or natural fiber can be provided to cover glass ampoule 37. In one form, the protective cover is formed from a cylinder or tube-like flexible non-woven polyester fabric jacket 38 that is crimped together and closed at both ends. A breathable material or polymer can be provided to cover the elongated glass ampoule 37 and fabric jacket 38. In a preferred form this breathable cover is formed from a cylinder or tube-like flexible micro porous polyethylene membrane or film 39 that is crimped together and closed at both ends.

Polyethylene film 39 lowers the rate of vaporization of the vaporizable material 35 when glass ampoule 37 is broken. By lowering the rate of vaporization, the polyethylene film 39 regulates the rate of vaporization of the vaporizable material 35 when it is exposed to the surrounding environment. Different ampoules may have different rates of vaporization due to different packaging and/or different vaporizable materials or perfumes. Providing ampoules with different vaporization rates is another way to control the life span of a group of cartridges 36. Films of different porosity may be selected and used to control or optimize the rate of vaporization, a valuable commercial feature. The fabric jacket 38 protects polyethylene film 39 from any rough or abrasive surfaces on glass ampoule 37, especially after the glass ampoule 37 is broken. Fabric jacket 38 also helps keep any pieces of the broken glass ampoule 37 together to allow for easy removal of the spent cartridge 36 from reusable magazine 40. If the vaporizable material 35 is a liquid or a sol, fabric jacket 38 can soak up the liquid like a sponge to become impregnated with the vaporizable material 35 in the liquid form, thereby preventing the liquid from draining out of magazine 40.

In another form, the vaporizable material 35 can comprise a gel-like air freshening material surrounded by a substantially impermeable gel encapsulation, similar to the gel encapsulation used to make pills containing liquids like vitamin E or cod liver oil. In this case fabric jacket 38 can be easily omitted since the gel encapsulation is not likely to have any abrasive surfaces. The gel encapsulation can be crushed, broken, dissolved, or otherwise changed to a condition in which the vaporizable material escapes to the atmosphere.

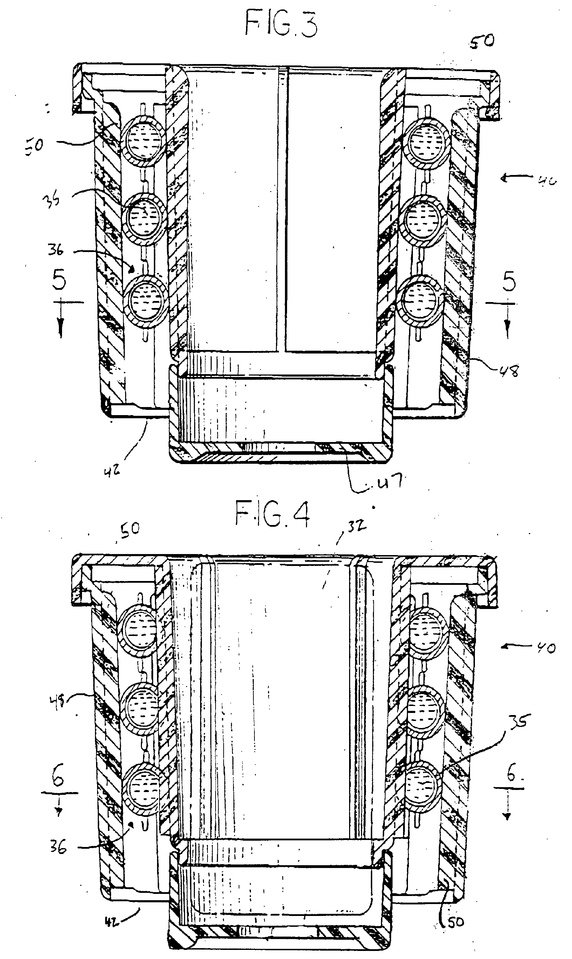

Referring to FIGS. 2-12, reusable magazine 40 is preferably circular in cross section, has an open upper end 41 facing motor and fan assembly 30, and is supported within deodorizing device 10 so that the reusable magazine 40 is in a position adjacent to the discharge of air from the fan 34. The discharge end 42 of the reusable magazine 40 includes a plurality of openings 43 for permitting free flow of air over and about the vaporizable material 35 contained therein.

A well 45, formed by a concentric inner wall 46, is in the magazine and cooperates with a base ring 47 attached to the inner wall 46 to form a battery chamber 45 for supporting and positioning the battery 32 concentrically within the reusable magazine 40. The well or battery chamber 45 is sized to permit easy insertion and removal of the battery 32 from the chamber for replacement when necessary.

The space between the outer surface of the inner wall 46 and the inner surface of an outer wall 48 of the reusable magazine 40 forms a cartridge receiving compartment that is used to hold the quantity of vaporizable material 35, and properly position this material in the air stream emitted by, or drawn by, the fan 34.

To this end, the inner face of the outer wall 48 includes a plurality of circumferentially spaced rib portions, such as cartridge retaining ribs 49, which retain the replaceable breakable air freshening cartridges 36 between a pair of retaining rib portions 49 and ensure its proper position in the air flow. In a preferred form, the inner face of the outer wall 48 also includes a smaller rib portion, such as cartridge positioning ribs 50, intermediate each pair of retaining rib portions 49 that help ensure the proper position of breakable cartridges 36. As shown in FIGS. 2-6, outer face of the inner wall 46 also contains a plurality of circumferentially spaced rib portions, such as cartridge breaking ribs 52.

In operation, the breakable cartridges 36 containing vaporizable material 35 are inserted into a reusable magazine 40. In a preferred form, multiple elongated breakable cartridges 36 are stacked one on top of each other and have each end 36a, 36b along its length adjacent a different rib portion retaining. When elongated cartridges 36 are properly positioned, positioning ribs 50 are preferably located approximately midway between each end 36a, 36b of elongated cartridge 36. Then battery 32 is inserted in the battery chamber 45 which helps stiffen the interior wall 46 and breaking ribs 52.

Breaking ribs 52 are moved relative to retaining ribs 49 and positioning ribs 50 by gripping and twisting sleeve portion 60 and insert portion 62 (see FIG. 2) in opposite directions. Outer wall 48 along with open upper end 41 or base ring 47 provide convenient places to grip. Breaking rib 52 preferably contacts glass ampoule 37 approximately midway along its length, such as midway between each end 36a, 36b of elongated cartridge 36, thereby exerting a force approximately opposite positioning rib 50 that breaks glass ampoule 37. Positioning rib 50 and breaking rib 52 thereby cooperate to exert a force at a weak portion of the depicted elongated glass ampoule 37.

The magazine 40 and battery 32 are inserted into the closure section 14. The base ring 47 of reusable magazine 40 engages the lowermost portion of the bracket 31 which functions to electrically couple one of the terminals of the battery 32 in an electrical circuit for energizing the fan motor 33. The opposite terminal for the battery 32 will be engaged by the other portion of bracket 31 which electrically couples the battery for completing the circuit to energize the motor 33 and initiate air movement. The fan motor 33 will then run until such time as it is necessary to replace the battery power source.

However, when replacing a discharged battery 32, if it is found that vaporizable material 35 is still contained within the reusable magazine 40, the battery 32 may be replaced by merely removing the reusable magazine 40 and inserting a fresh battery in place of the one that has been discharged. In this manner, the entire reusable magazine 40 does not need to be replaced merely because the battery 32 has been discharged. Similarly, if it is found that the vaporizable material 35 has been expended, the expended replaceable cartridges 36 may merely be replaced with new replaceable cartridges 36. Alternatively, the reusable magazine 40 can be replaced along with the cartridges 36 in order to minimize exposure to vaporizable material 35, especially for people with allergies or extremely sensitive skin.

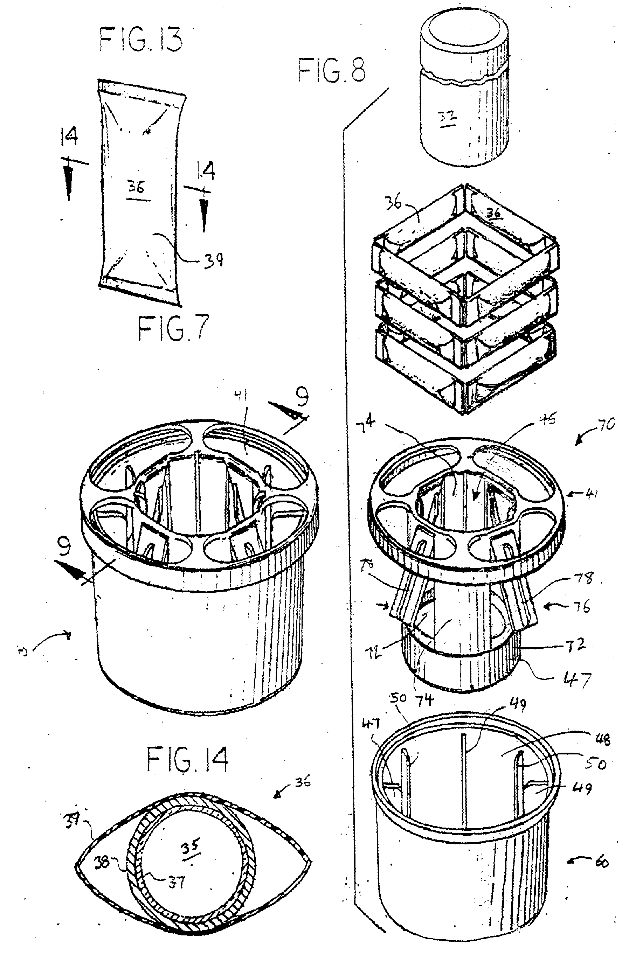

In an alternative form, best shown in FIGS. 7-12, the reusable magazine 40 is altered slightly. Essentially, the reusable magazine 40 substitutes a modified insert 70 in place of the insert 62 of the embodiment best shown in FIGS. 2-6. As depicted in the drawings, insert 70 is similar to, and has a number of the same components as, the insert 62 described above. The main differences are those structural and operational differences indicated in the drawings and described below.

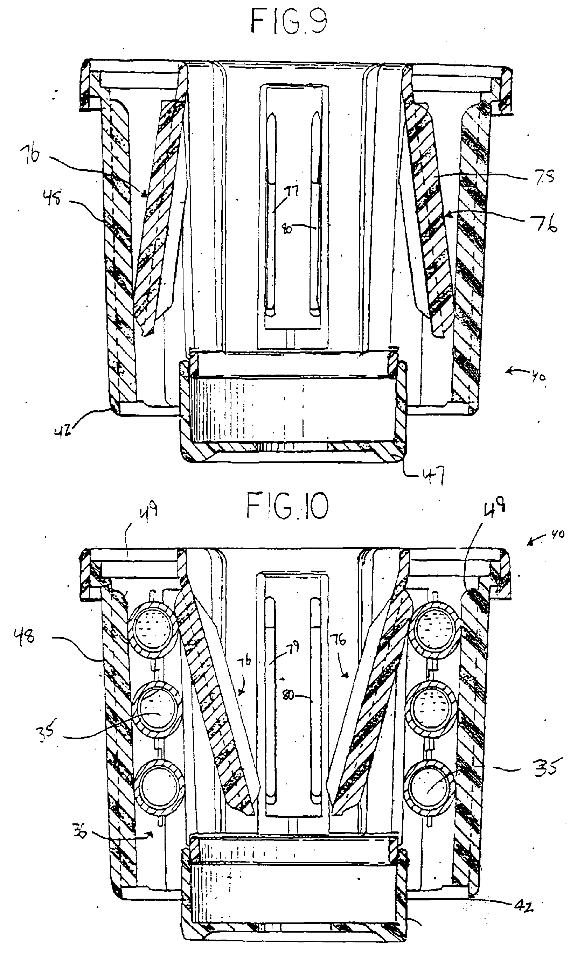

A plurality of openings 72 are defined by inner wall 74 of insert 70. A living hinge 76 is hingeably coupled to inner wall 74 at the top of opening 72 and preferably moveable from a position largely inside insert 70 (see FIG. 10) through opening 72 to a position outside insert 70 (see FIGS. 8 and 10) and generally towards ribs 49 and 50. Living hinge 76 has a breaking rib portion 78 on the side of the rib facing the exterior of inner wall 74. A battery contact portion, such as a pair of battery engaging rib portions 79 and 80, are provided on the opposite side of living hinge 76 and facing the interior of inner wall 74.

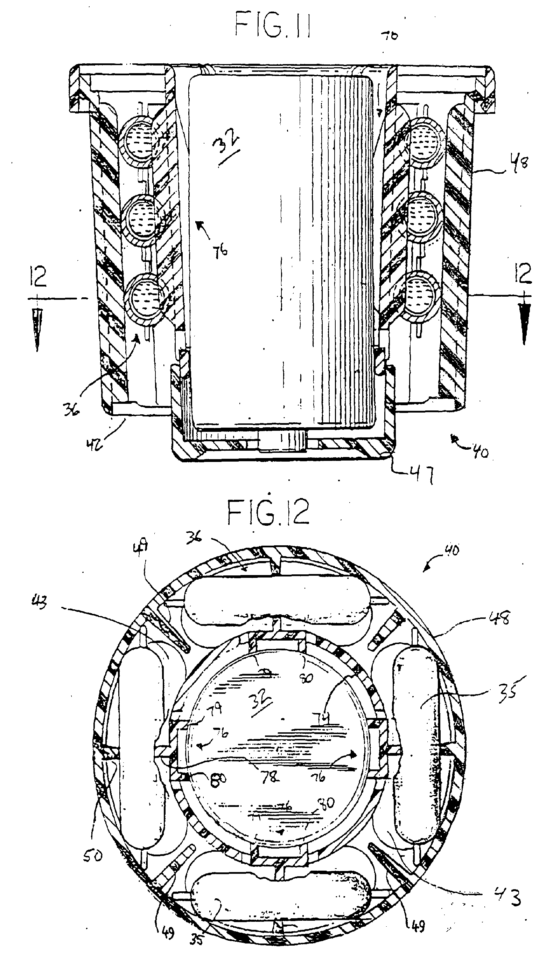

This embodiment operates largely the same as described for the previous embodiment. However, the replaceable cartridges 36 are broken in a slightly different manner. The replaceable cartridges 36 are preferably loaded into reusable magazine 40 in the same manner as described above. When cartridges 36 are properly positioned, breaking ribs 78 are preferably located approximately midway between each end 36a, 36b of elongated cartridge 36. As battery 32 is inserted in the battery chamber 45, battery 32 contacts battery engaging rib portions 79 and 80 causing living hinge 76 and breaking rib portion 78 to move towards the replaceable cartridges 36 (see FIGS. 11 and 12). Breaking rib 78 preferably contacts glass ampoule 37 approximately midway along its length, approximately midway between each end 36a, 36b of replaceable cartridge 36, thereby exerting a force approximately opposite positioning rib 50 that breaks glass ampoule 37 (see FIG. 12). Positioning rib 50 and breaking rib 78 thereby cooperate to exert a force at a weak portion of the depicted elongated glass ampoule 37. The reusable magazine 40 and the battery 32 are inserted into closure section 14 as previously described.

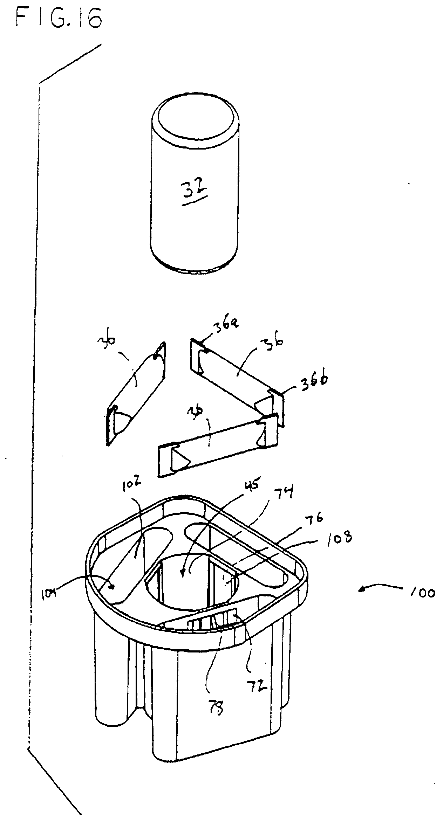

In an alternative form shown in FIGS. 15 and 16, a reusable magazine 100 is somewhat altered, but quite similar to the embodiment shown in FIGS. 7-12. Essentially, the reusable magazine 100 is of a one piece, preferably molded, construction. In this form, sleeve 60 and insert 70 are essentially combined into a unitary magazine 100. As depicted in the drawings, magazine 100 is similar to, and has a number of the same components as, the embodiment of FIGS. 7-12. The main differences are those structural and operational differences indicated in the drawings and described below.

A plurality of openings 72 are defined by inner wall 74 of magazine 100. A living hinge 76 is hingeably coupled to inner wall 74 at the top of opening 72 and preferably moveable from a position largely inside inner wall 74 through opening 72 to a position largely outside inner wall 74 and generally towards outerwall 102 of cartridge receiving compartment 104. In this embodiment, cartridge receiving compartment 104 does not necessarily have the variety of ribs that position or hold the cartridges in place. Instead, the cartridge receiving compartment 104 can be sized to accept one or more cartridges 36 and hold them in place. Living hinge 76 has a breaking rib portion 78 on the side of the rib facing the exterior of outer wall 102 of cartridge receiving compartment 104. A battery engaging portion 108 is provided on the opposite side of living hinge 76 and faces the interior of battery receiving compartment 45.

This embodiment operates largely the same as described for the previous embodiment. The replaceable cartridges 36 are broken in a very similar manner. The replaceable cartridges 36 are preferably loaded into reusable magazine 100 in a similar manner as described above. When cartridges 36 are properly positioned, breaking ribs 78 are preferably located approximately midway between each end 36a, 36b of elongated cartridge 36. As battery 32 is inserted in the battery chamber 45, battery 32 contacts battery engaging portions 108 causing living hinge 76 and breaking rib portion 78 to move towards the replaceable cartridges 36. Breaking rib 78 preferably contacts glass ampoule 37 approximately midway along its length, approximately midway between each end 36a, 36b of replaceable cartridge 36. Breaking rib 78 thereby exerts a force at a weak portion of the depicted elongated glass ampoule 37. The reusable magazine 40 and the battery 32 are inserted into closure section 14 as previously described. If desired, a positioning rib can be provided opposite breaking rib 78 as previously described in the previous embodiment.

While particular embodiments of the present invention have been shown and described, it will be appreciated by those skilled in the art that changes and modifications may be made without departing from the invention in its broader aspects. Therefore, the aim in the appended claims is to cover all such changes and modifications as fall within the true spirit and scope of the invention. The matter set forth in the foregoing description and accompanying drawings is offered by way of illustration only and not as a limitation. The actual scope of the invention is intended to be defined in the following claims when viewed in their proper perspective based on the prior art.

## Claims

1. A replaceable air freshener or deodorizer cartridge (36) comprising an air freshening or deodorizing material (35) and a container (37) encapsulating the material (35), characterised in that the container (37) is transformable from an impermeable state where the container is substantially impermeable to the material (35), to a permeable state where the container is permeable to the material (35).

2. A cartridge according to claim 1, characterised in that it further comprises a flexible breathable cover (39) surrounding the container (37), wherein the breathable cover limits a vaporization rate of the material (35) when the container (37) is in the permeable state.

3. A cartridge according to claim 1 or 2, characterised in that the container is a breakable container (37), the breakable container being unbroken in the impermeable state and broken in the permeable state.

4. A cartridge according to claim 3, characterised in that the breakable container is comprised of glass.

5. A cartridge according to claim 3 or 4, characterised in that it further comprises a flexible protective cover (38) surrounding the breakable container (37), wherein the protective cover (38) traps substantially all of the breakable container (37) when the container is in the permeable state and protects the breathable cover (39) from abrasive surfaces of the breakable container.

6. A cartridge according to any one of the preceding claims, characterised in that the air freshening or deodorizing material (35) comprises a gel.

7. A cartridge according to claim 2 or any one of claims 3 to 6 when dependent on claim 2, characterised in that the breathable cover (39) comprises a microporous film.

8. A cartridge according to claim 7, characterised in that the microporous film comprises a polymer film.

9. A cartridge according to claim 8, characterised in that the polymer film comprises polyethylene.

10. A cartridge according to claim 5 or any one of claims 6 to 9 when dependent on claim 5, wherein the protective cover (38) comprises a fabric jacket.

11. A cartridge according to claim 10, characterised in that the fabric jacket is comprised of a non-woven fabric.

12. A cartridge according to claim 11, characterised in that the non-woven fabric is comprised of a polyester.

13. A cartridge according to claim 5 or any one of claims 6 to 12 when dependent on claim 5, characterised in that the protective cover (38) comprises a fabric cylinder having two ends, each end being crimped together and closed.

14. An air freshening or deodorizing magazine (40) for use within a self-contained air freshener apparatus, the magazine comprising: a cartridge receiving compartment having an outer wall (48) and adapted to receive an associated breakable air freshening or deodorizing cartridge (36) removably inserted therein; and a cartridge breaking structure (76) disposed within the cartridge receiving compartment and moveable relative to the cartridge receiving compartment; wherein when the cartridge breaking structure (76) moves relative to the cartridge receiving compartment, the cartridge breaking structure engages and exerts a breaking force on the associated breakable cartridge.

15. A magazine according to claim 14, characterised in that it further comprises a cartridge retaining structure (49, 50) disposed within the cartridge receiving compartment, the cartridge breaking structure being moveable relative to the cartridge retaining structure, wherein the cartridge retaining structure (49,50) and cartridge breaking structure (76) cooperate with each other to retain and position the associated breakable cartridge, such that when the cartridge breaking structure moves relative to the cartridge retaining structure, the cartridge breaking structure engages and exerts a breaking force on the associated breakable cartridge.

16. A magazine according to claim 15, characterised in that it further comprises a hinge (76) coupled to the cartridge breaking structure, wherein the hinge (76) is movable to engage and exert a breaking force on the associated breakable cartridge.

17. A magazine according to claim 16, characterised in that it further comprises a battery engaging surface (79, 80) coupled to the hinge (76) and a battery chamber (45) coupled to the hinge, wherein when an associated battery (32) is inserted in the battery chamber (45), the battery engages the battery engaging surface and pivots the hinge (76) such that the cartridge breaking structure engages and exerts a breaking force on the associated breakable cartridge.

18. A magazine according to any one of claims 14 to 17, characterised in that the cartridge receiving compartment is adapted to receive a plurality of associated breakable cartridges.

19. A magazine according to claim 18, characterised in that it further comprises one or more breakable cartridges inserted in the cartridge receiving compartment.

20. A magazine according to claim 19, characterised in that said one or more breakable cartridges comprise at least two different scented cartridges.

21. A magazine according to any one of claims 14 to 20, characterised in that it further comprises a battery (32) inserted in the battery chamber (45).

22. A replaceable air freshening or deodorizing magazine for use within a self-contained air freshener apparatus, the magazine comprising: an outer wall (48) and an inner wall (46) defining a compartment; a retaining structure (49,50) located within the compartment and coupled to one of an exterior surface of the inner wall (46) and an interior surface of the outer wall (48), the retaining structure being structured to retain an associated breakable air freshening cartridge (36) removably insertable in the compartment; and a cartridge breaking surface located within the compartment and coupled to the other of said one of the exterior surface of the inner wall and an interior surface of the outer wall, the cartridge breaking surface being rotatable relative to the retaining surface; wherein when the 5 cartridge breaking surface rotates relative to the retaining structure, the cartridge breaking surface engages and exerts a breaking force on the associated breakable air freshening cartridge inserted in the compartment.

23. A magazine according to claim 22, characterised in that the retaining structure (49,50) protrudes from an interior surface of the outer wall (48) and the cartridge breaking surface (52) protrudes from an exterior surface of the inner wall (46).

24. A magazine according to claim 22 or 23, characterised in that the retaining structure comprises a plurality of retaining ribs (49) spaced about an interior surface of said outer wall (48) and extending inwardly toward the exterior surface of said inner wall (46), each pair of retaining ribs (49) being adapted to retain the associated breakable air freshening cartridge inserted therein.

25. A magazine according to claim 23 or 24, characterised in that the cartridge breaking surface comprises at least one breaking rib (52) located on an exterior surface of said inner wall (46) and extending inwardly toward the interior surface of said outer wall (48).

26. A magazine according to claim 25, characterised in that each pair of retaining ribs (49) is adapted to retain an associated elongated breakable air freshening cartridge (36) having a first end and a second end and wherein the cartridge breaking surface (52) engages the associated elongated cartridge at a point intermediate the first and second ends.

27. A magazine according to claim 26, characterised in that the breaking rib (52) engages the associated elongated cartridge at a point approximately equidistant from the first and second ends.

28. A magazine according to any one of claims 23 to 27, characterised in that it further comprises a cartridge positioning structure (50) located on the interior surface of said outer wall (48) and wherein the cartridge positioning structure engages the associated elongated cartridge at a point intermediate the first and second ends.

29. A magazine according to claim 28, characterised in that the cartridge positioning structure comprises a positioning rib (52) extending inwardly toward the exterior surface of said inner wall and wherein the positioning rib engages the associated elongated cartridge at a point approximately equidistant from the first and second ends.

30. A magazine according to claim 28 or 29, characterised in that it further comprises at least one elongated breakable air freshener or deodorizer cartridge positioned within the cartridge retaining structure.

31. A self-contained air freshening or deodorizing apparatus comprising a replaceable cartridge (40) containing a quantity of a product (35) capable of being vaporized to release the product in vapour form, and air movement device (30), the replaceable cartridge (40) being positionable within the apparatus in a flow of air movement generated by the air movement device (30), characterised in that the replaceable air freshening or deodorizing cartridge (40) comprises a breakable cartridge (37) encapsulating an air freshening or deodorizing liquid, the cartridge being transformable from an impermeable state where the cartridge is substantially impermeable to the liquid, to a permeable state where the cartridge is permeable to the liquid.

32. An apparatus according to claim 31, characterised in that the replaceable cartridge (40) further comprises a flexible breathable cover (39) surrounding the breakable cartridge (37), wherein the breathable cover limits a vaporization rate of the liquid when the cartridge is in the permeable state.

33. An apparatus according to claim 31 or 32, characterised in that the replaceable cartridge further comprises a flexible protective cover (38) surrounding the breakable cartridge (37), wherein the protective cover traps substantially all of the breakable cartridge when the cartridge is in the permeable state and protects the breathable cover from abrasive surfaces of the breakable cartridge.
